(19) **Europäisches Patentamt / European Patent Office / Office européen des brevets**

(11) **EP 2 020 248 B1**

(12) **EUROPEAN PATENT SPECIFICATION**

(45) Date of publication and mention of the grant of the patent:
**30.03.2011 Bulletin 2011/13**

(21) Application number: **04822649.2**

(22) Date of filing: **18.11.2004**

(51) Int Cl.:
**A61M 5/00** *(2006.01)*      **A61M 5/142** *(2006.01)*

(86) International application number:
**PCT/JP2004/017154**

(87) International publication number:
**WO 2006/054343 (26.05.2006 Gazette 2006/21)**

(54) **CARDIAC DISEASE TREATMENT SYSTEM**

SYSTEM ZUR BEHANDLUNG VON HERZKRANKHEITEN

SYSTEME DE TRAITEMENT D'UNE MALADIE CARDIAQUE

(84) Designated Contracting States:
**AT BE BG CH CY CZ DE DK EE ES FI FR GB GR HU IE IS IT LI LU MC NL PL PT RO SE SI SK TR**

(43) Date of publication of application:
**04.02.2009 Bulletin 2009/06**

(73) Proprietor: **Japan Health Sciences Foundation Tokyo 103-0001 (JP)**

(72) Inventors:
- **UEMURA, Kazunori**
  **Toyonaka-shi**
  **Osaka 5600005 (JP)**
- **KAMIYA, Atsunori**
  **Suita-shi,**
  **Osaka 5650875 (JP)**
- **SUGIMACHI, Masaru**
  **Suita-shi,**
  **Osaka 5650873 (JP)**

- **SUNAGAWA, Kenji**
  **Fukuoka-shi**
  **Fukuoka 8190373 (JP)**

(74) Representative: **Gemmell, Peter Alan et al Dummett Copp LLP**
**25 The Square**
**Martlesham Heath**
**Ipswich IP5 3SL (GB)**

(56) References cited:
EP-A- 0 297 675      JP-A- 01 305 962
JP-A- 2000 507 129   US-A- 5 368 040
US-A1- 2003 149 450  US-A1- 2003 199 813

- UEMURA, SUGIMACHI, KAWADA, KAMIYA, JIN, KASHIHARA, SUNAGAWA: "A novel framework of circulatory equilibrium" AM J PHYSIOL HEART CIRC PHYSIOL, vol. 286, no. 6, 5 February 2004 (2004-02-05), pages H2376-H2385, XP002507894

**Description**

**BACKGROUND OF THE INVENTION**

**Field of the Invention**

The present invention relates to a cardiac disease treatment system. More particularly, the present invention provides a system for treating cardiac diseases by accurately diagnosing the functional causes of abnormalities related to cardiac diseases and then automatically administering drugs according to the diagnosis.

**Description of the Background Art**

Cardiac diseases are effectively treated by an emergency care by specialized cardiologists during the acute phase. However, only 3% of all physicians are specialized in treating cardiac diseases, which is not sufficient.

Thus, it is required that general physicians should be capable of dealing with cardiac diseases at a comparable level to specialized cardiologists.

It has been difficult for general physicians to reach such a level. Therefore, there is a need for the development of a cardiac disease treatment system that provides general physicians with therapeutic skills comparable to those of specialized cardiologists.

As one solution to the above-mentioned problem, there has been created a cardiac disease treatment system disclosed in Tokuhyou 2000-507129 that automatically adjusts the intravenous administration of cardiovascular drugs. The system disclosed in Tokuhyou 2000-507129 is used to automatically control the intravenous administration of cardiovascular drugs (e.g., vasodilators) to a patient for stabilization of drug concentration in blood, for example, during anesthesia.

The system described in Tokuhyou 2000-507129, however, aims at control of drug concentration in blood or the pharmacokinetics of drugs in patients, but not at improvement of the hemodynamic parameters or the organ systems of patients. Also, the system is applicable only to patients under anesthesia. Therefore the system does not solve the above problems.

Since the basic circulatory equilibrium theory utilizing cardiac output curve and venous return curve was established by Guyton et al. in the 1950's, studies for applying this theory for treatment of cardiac diseases have continued (see "Determination of cardiac output by equating venous return curves with cardiac response curves" Physiol Rev 35: 123-129,1955).

However, redistribution of blood between the pulmonary and systemic circulations is not taken into account in the Guyton's basic circulatory equilibrium theory. The theory does not enable the prediction of left atrial pressure (pulmonary arterial wedge pressure), which affects the prognosis, not allowing accurate treatment.

There are many conventional devices capable of measuring cardiac output, left atrial pressure, arterial blood pressure, and heart rate to display abnormalities in hemodynamics. No systems are available that diagnoses the internal functional structure of circulatory system by determining whether the abnormalities in hemodynamic parameters result from pumping ability or effective circulating blood volume (stressed blood volume).

Since it is not possible for these conventional devices to diagnose the functional cause of abnormalities indicated by abnormal measured values in hemodynamics as described above, the selection of a treatment method has been entirely dependent on the experience of specialized cardiologists.

US2003/0199813 and US2003/0149450 are prior art publications which disclose closed-loop drug administration systems for treatment of cardiac disease, taking into account blood pressure, heart rate and cardiac output. "A novel frame work of circulatory equilibrium" (Am) Physiol Heart Circ Physiol, vol.286(6), H2376-H2385, Feb 5 2004) discusses basic considerations about cardiac output, atrial pressure, blood volume and vascular resistance.

**DISCLOSURE OF THE INVENTION**

**Problems to be solved**

Considering the above-mentioned problems, one object of the present invention is to provide a cardiac disease treatment system that analyzes the hemodynamics of a patient, specifies the functional cause of abnormalities related to cardiac diseases, and automatically administers drugs for treating cardiac diseases according to the diagnosis.

**Means for solving the problems**

The invention defined in Claim 1 relates to a cardiac disease treatment system comprising: an input means inputting a cardiac output value, a left atrial pressure value, a right atrial pressure value, and arterial blood pressure value of a patient; a first calculation means calculating a pumping ability value of the left heart or a pumping ability value of the right heart from the cardiac output value, the left atrial pressure value, and the right atrial pressure value which are input from the input means; a first comparison means comparing calculated values of the pumping ability value of the left heart and/or the pumping ability value of the right heart and target pumping ability values; and a first dosing device administering drugs to the patient according to comparison results in the first comparison means; a second calculation means calculating an effective circulating blood volume value from the cardiac output value, the left atrial pressure value, and the right atrial pressure value which are input from the input means; a second comparison means comparing the effective circulating blood volume value calculated by the second calculation means and a target effective circulating blood volume value; a second dosing device administering drugs to the patient according to the comparison result in the second comparison means; a third calculation means calculating a vascular resistance value from the cardiac output value, the right atrial pressure value, and the arterial blood pressure value which are input by the input means; a third comparison means comparing the vascular resistance value calculated by the third calculation means and a target vascular resistance value; and a third dosing device administering drugs to the patient according to the comparison result in the third comparison means.

Claim 2 relates to the cardiac disease treatment system wherein the first calculation means uses equation 1 and/or equation 2 to calculate the pumping ability value of the left heart and/or the pumping ability value of the right heart from the cardiac output value, the left atrial pressure value, and/or the right atrial pressure value input by the input means; the second calculation means uses equation 5 to calculate the effective circulating blood volume value from the cardiac output value, the left atrial pressure value, and the right atrial pressure value input by the input means; and the third calculation means uses equation 7 to calculate the vascular resistance value from the cardiac output value, the right atrial pressure value, and the arterial blood pressure value input by the input means.

## Equation 1

(cardiac output value)

= (pumping ability value of the left heart) x {Log ((left atrial pressure value) - A) + B}

wherein A and B are constants;

## Equation 2

(cardiac output value)

= (pumping ability value of the right heart) x {Log ((right atrial pressure value) - C) + D}

wherein C and D are constants;

## Equation 5

$$(\text{cardiac output value}) = \frac{1}{E} (\text{effective circulating blood volume value})$$
$$- F (\text{right atrial pressure value}) - G (\text{left atrial pressure value})$$

wherein E, F, and G are constants;

## Equation 7

$$\text{(vascular resistance value)} = \frac{\{\text{(arterial blood pressure value)} - \text{(right atrial pressure value)} - H\}}{\text{(cardiac output value)}}$$

wherein H is a constant.

Claim 3 relates to the cardiac disease treatment system further comprising: a first target decision means calculating a target pumping ability value of the left heart and/or a target pumping ability value of the right heart from the target cardiac output value, the target left atrial pressure value, and/or the target right atrial pressure value; a second target decision means calculating a target effective circulating blood volume value from the target cardiac output value, the target left atrial pressure value, and the target right atrial pressure value; and a third target decision means calculating a target vascular resistance value from the target cardiac output value, the target right atrial pressure value, and the target arterial blood pressure value.

Claim 4 relates to the cardiac disease treatment system wherein the first target decision means uses equation 3 and equation 4 to calculate the target pumping ability value of the left heart and/or the pumping ability value of the right heart from the target cardiac output value, the target left atrial pressure value, and/or the right atrial pressure value; wherein the second target decision means uses equation 6 to calculate the target effective circulating blood volume value from the target cardiac output value, the target left atrial pressure value, and the target right atrial pressure value; and wherein the third target decision means uses equation 8 to calculate the target vascular resistance value from the target cardiac output value, the target right atrial pressure value, and the target arterial blood pressure value:

## Equation 3

$$\text{(target pumping ability value of the left heart)} = \frac{\text{(target cardiac output value)}}{\{\text{Log}((\text{target left atrial pressure value}) - A) + B\}}$$

wherein A and B are constants;

## Equation 4

$$\text{(target pumping ability value of the right heart)} = \frac{(\text{target cardiac output value})}{\{\text{Log}((\text{target right atrial pressure value}) - C) + D\}}$$

wherein C and D are constants;

## Equation 6

$$\text{(target effective circulating blood volume value)} = \{\text{(target cardiac output value)} + F(\text{target right atrial pressure value}) + G(\text{target left atrial pressure value})\} \times E$$

wherein E, F, and G are constants;

## Equation 8

$$\text{(target vascular resistance value)} = \frac{\{\text{(target arterial blood pressure value)} - \text{(target right atrial pressure value)} - H\}}{\text{(target cardiac output value)}}$$

wherein H is a constant.

Claim 5 relates to the cardiac disease treatment system further comprising: a display means continuously displaying each of the calculated values calculated by the calculation means in chronological order.

Claim 6 relates to the cardiac disease treatment system wherein the cardiac output value is measured by a Swan-Ganz catheter or calculated from a diastolic time constant of arterial blood pressure waveform.

Claim 7 relates to the cardiac disease treatment system wherein the left atrial pressure value is directly measured by a catheter or continuously estimated from diastolic pressure values of pulmonary capillary wedge pressure or pulmonary arterial pressure which are measured by a Swan-Ganz catheter.

## **Effects of the invention**

According to Claim 1, a cardiac disease treatment system calculates a pumping ability value of the left heart or the right heart from cardiac output and left atrial pressure and/or right atrial pressure. The cardiac disease treatment system then compares, the calculated pumping ability value of the left heart and/or the right heart with target pumping ability values and diagnoses the abnormalities in pumping ability to administer drugs according to the diagnosis.

Administration of drugs according to the comparison results between the pumping ability value of the left heart or the right heart and the target pumping ability values secures that abnormal pumping ability is accurately improved to be in a normal state during a treatment to a patient.

The cardiac treatment system also calculates an effective circulating blood volume value of the patient from cardiac output, left atrial pressure, and right atrial pressure of a patient. The cardiac treatment system then compares the calculated effective circulating blood volume value with the target effective circulating blood volume value to administer drugs according to the comparison result.

The administration according to the comparison between the calculated effective circulating blood volume value and the target effective circulating blood volume value secures that the abnormal effective circulating blood volume is corrected to be in a normal state during a treatment to the patient.

The cardiac disease treatment system further calculates a vascular resistance value from input values of cardiac output, right atrial pressure, and arterial blood pressure of a patient and compares the calculated vascular resistance value and the target vascular resistance value to administer drugs according to the comparison result.

The administration of drugs according to the comparison result between the calculated vascular resistance value and the target vascular resistance value secures that the abnormal vascular resistance value is accurately corrected to be in a normal range.

According to Claim 2, the cardiac treatment system uses Equation 1 and Equation 2 in order to calculate pumping ability more minutely and clearly Adjustment of constants A to D allows the pumping ability values calculated for various patients to be corrected.

Correction of the calculated pumping ability values for each patient enables more accurate calculation of the pumping ability for more accurate treatment to each patient.

The cardiac disease treatment system also uses Equation 5 to calculate the effective circulating blood volume more minutely and clearly. Adjustment of constants E to G allows the effective circulating blood volume values calculated for various patients to be corrected.

Correction of the calculated effective circulating blood volume value for each patient enables more accurate calculation of the effective circulating blood volume for more accurate treatment to each patient.

The cardiac disease treatment system uses Equation 7 to calculate the vascular resistance value more minutely and clearly Constant H in equation 7 is employed for correcting the non-linearity of vascular resistance. Adjustment of constant H enables normal operation of the system to be maintained even in a subject with a highly non-linear vascular resistance.

According to Claim 3, the cardiac treatment system uses a target cardiac output value, a target left atrial pressure value and/or a target right atrial pressure value to calculate target pumping ability values including a target pumping ability value of the left heart and/or a target pumping ability value of the right heart in a simple way.

The cardiac disease treatment system calculates the target effective circulating blood volume value from the target cardiac output value, the target left atrial pressure value, and the target right atrial pressure value in a simple way.

The cardiac disease treatment system calculates the target vascular resistance value from the target cardiac output value, the target right atrial pressure value, the target arterial blood pressure value in a simple way.

According to Claim 4, the cardiac treatment system uses Equation 3 and Equation 4 to more appropriately calculate the target pumping ability values.

The cardiac disease treatment system uses Equation 6 to more appropriately calculate the target effective circulating blood volume value, and the cardiac disease treatment system uses Equation 8 to more appropriately calculate the target vascular resistance value.

According to Claim 5, the cardiac disease treatment system continuously displays each value of pumping ability of the left heart and/or the right heart, effective circulating blood volume, and vascular resistance. This prevents a chronological change in patient conditions from being ignored and enables reliable diagnosis on a patient. The cardiac disease treatment system also displays the effect of drug administration on patient conditions.

According to Claims 6 and 7, cardiac output is measured by a Swan-Ganz catheter or calculated from a diastolic time constant of arterial blood pressure waveform whereas left atrial pressure is directly measured by a catheter or continuously estimated from diastolic pressure values of pulmonary capillary wedge pressure or pulmonary arterial pressure which are measured by a Swan-Ganz catheter. This results in a highly accurate cardiac disease treatment system.

## BRIEF DESCRIPTION OF THE DRAWINGS

Fig. 1 is a schematic block diagram of the cardiac disease treatment system according to the present invention;

Fig. 2 is a schematic view of a heart;

Fig. 3 shows one embodiment of cardiac output curve in a three dimensional coordinate diagram of cardiac output, left atrial pressure, and right atrial pressure;

Fig. 4 shows one embodiment of venous return surface in a three dimensional coordinate diagram of cardiac output, left atrial pressure, and right atrial pressure;

Fig. 5 shows one embodiment of a cardiac output curve and venous return surface in a three dimensional coordinate diagram of cardiac output, left atrial pressure, and right atrial pressure;

Fig. 6 is a flowchart illustrating use of the cardiac disease treatment system according to the present invention;

Fig. 7 shows changes in the arterial blood pressure value, the right atrial pressure value, the left atrial pressure value, and the cardiac output value with use of the cardiac disease treatment system according to the present invention; and

Fig. 8 shows effective circulating blood volume, pumping ability of the left heart, pumping ability of the right heart, vascular resistance, and the change in the dose of drugs with horizontal lines in each trace displaying a target pumping ability value of the left heart, a target effective circulating blood volume value, and a target vascular resistance value.

## PREFERRED EMBODIMENTS OF THE INVENTION

Hereinafter, preferred embodiments of the present invention will be described.

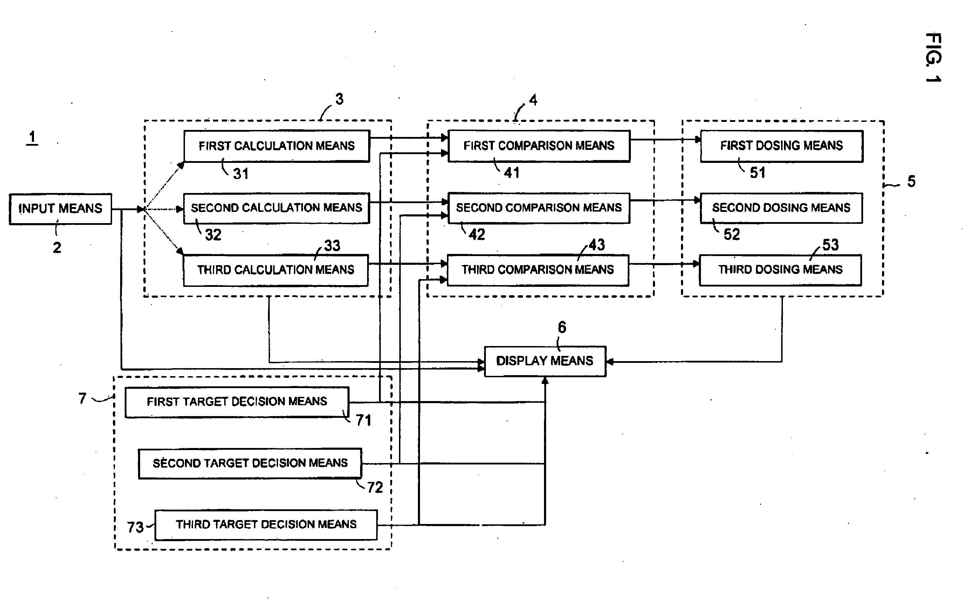

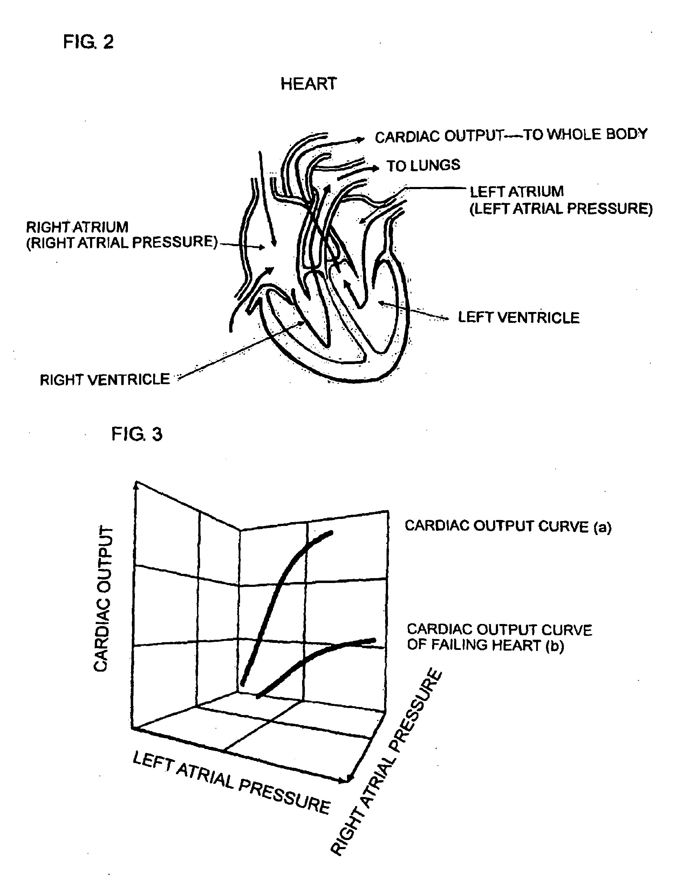

Fig. 1 is a block diagram schematically showing a cardiac disease treatment system according to the present invention. Fig. 2 is a schematic view of a heart. Cardiac disease treatment system (1) in the present invention utilizes cardiac output (CO) values, left atrial pressure (Pla) values, and right atrial pressure (Pra) values shown in Fig. 2, as well as arterial blood pressure (AP) values (not shown).

Cardiac disease treatment system (1) is used to determine the functional cause of abnormality in the cardiovascular system based on a hemodynamic abnormality (e.g., reduction in CO (peripheral circulatory failure), elevation in Pla (pulmonary congestion), and arterial blood pressure (increase and decrease)) to administer appropriate therapeutic agents.

Cardiac disease treatment system (1) includes an input means (2), a calculation means (3), a comparison means (4), a dosing means (5), a display means (6), and a target decision means (7).

Input means (2) inputs measurement values (numeric data) of the hemodynamics in a patient to calculation means (3) (described below). Input means (2) is configured to input measurement values of AP, CO, Pra, and Pla.

Input means (2) may be any configuration that is capable of inputting numeral data to calculation means (3). Input means (2) may be an input device (e.g., keyboard) that allows a person using cardiac disease treatment system (1) to input actual measurement values, or a measurement device that measures the hemodynamics in a patient and inputs the values directly to calculation means (3), though not limited to these. If cardiac disease treatment system (1) is constantly connected to a patient for drug administration in order to diagnose abnormalities related to cardiac diseases, cardiac disease treatment system (1) is preferably configured to allow the measurement values of the hemodynamics in a patient to be directly input to calculation means (3).

The hemodynamics numeric data (AP value and CO value, as well as Pra and Pla values) input by input means (2) are respectively obtained using a conventional measurement device. The measurement device may be a catheter placed in a peripheral artery (e.g., radial artery) for measuring AP and a Swan-Ganz catheter for measuring CO, Pla, and Pra, though not limited to these.

Cardiac disease treatment system (1) according to the present invention continuously obtains these measurement values and uses them for a continuous diagnosis of a patient. However, it is known to be impossible to continuously measure Pla and CO, although continuous measurement of AP and Pra is possible.

Diastolic pulmonary arterial pressure is used for continuously estimating Pla to obtain a continuous value.

As generally known, there is a linear relationship between Pla and diastolic pulmonary arterial pressure. Pla may be calculated from diastolic pulmonary arterial pressure based on an average correlation for multiple subjects. It is preferable to allow the average correlation (linear relationship) between diastolic pulmonary arterial pressure and Pla to be corrected according to heart rate, which causes a change in the correlation.

Diastolic time constant of peripheral arterial blood pressure waveform is used for estimating CO to obtain a continuous value.

CO may be, for example, calculated from diastolic time constant of peripheral arterial blood pressure waveform using a conventional method.

Target decision means (7) calculates target values of pumping ability (pumping ability of the left heart and/or the right heart), effective circulating blood volume, and vascular resistance. These target values are used as reference values by first to third comparison means (described below). Once target values of CO, Pla, Pra, and AP are input, target decision means (7) automatically figures out the target values of pumping ability (pumping ability of the left heart and/or the right heart), effective circulating blood volume, and vascular resistance. It is also possible for a user to input in advance these target values of pumping ability (pumping ability of the left heart and/or the right heart), effective circulating blood volume, and vascular resistance to the respective comparison means.

Target decision means (7) includes a first target decision means (71), a second target decision means (72), and a third target decision means (73). First to third target decision means (71-73) are processing units configured to calculate output values according to input values.

Target decision means (7) may be a single processing unit that plays roles of first to third target decision means (71-73) in performing computations. Alternatively, target decision means (7) may include three processing units respectively play roles of first to third target decision means (71-73) in performing computations.

First target decision means (71) calculates a target pumping ability value of the left heart from the target values of CO and Pla, and/or calculates a target pumping ability value of the right heart from the target values of CO and Pra.

First target decision means (71) may be configured to calculate both or either of the above-mentioned target pumping ability value of the left heart and/or the right heart.

In calculation of a target pumping ability value of the left heart by first target decision means (71), a target CO value and a target Pla value are substituted in the following Equation 9 to figure out the target pumping ability value of the .left heart value.

In Equation 9, constants A and B are user preset values that are adjustable for each patient. Adjustment of these values for each patient allows the calculated pumping ability value of the left heart to be corrected.

## Equation 9

$$\text{(target pumping ability value of the left heart)} = \text{(target CO value)} \Big/ \{\text{Log}((\text{target Pla value}) - \text{A}) + \text{B}\}$$

(where both A and B are constants)

First target decision means (71) calculates a target pumping ability value of the right heart using the following Equation 10 by a similar calculation to the above-described calculation of the target pumping ability value of the left heart. The target pumping ability value of the right heart is calculated by substituting the target CO value and the target Pra value in Equation 10.

In Equation 10, constants C and D are user preset values that are adjustable for each patient. Adjustment of these values for each patient allows the calculated pumping ability value of the right heart to be corrected.

## Equation 10

$$\text{(target pumping ability value of the right heart)} = \text{(target CO value)} \Big/ \{\text{Log}((\text{target Pra value}) - \text{C}) + \text{D}\}$$

(where both C and D are constants)

Target decision means (7) includes second target decision means (72), as described above.

Second target decision means (72) calculates a target effective circulating blood volume value from the target CO value, the target Pla value, and the target Pra value.

In calculation of the target effective circulating blood volume value by second target decision means (72), the target CO value, the target Pla value, and the target Pra value are substituted in the following Equation 11 to figure out the target effective circulating blood volume value.

In Equation 11, constants E, F, and G are user preset values that are adjustable for each patient. Adjustment of these values for each patient allows the calculated effective circulating blood volume value to be corrected.

### Equation 11

$$\text{(target effective circulating blood volume value)}$$
$$= \{(\text{target CO value}) + F(\text{target Pra value}) + G(\text{target Pla value})\} \times E$$

(where all of E, F, and G are constants)

Target decision means (7) includes third target decision means (73), as described above.

Third target decision means (73) calculates a target vascular resistance value from the target CO value, the target Pra value, and the target AP value.

In calculation of the target vascular resistance value by third target decision means (73), the target CO value, the target Pra value, and the target AP value are substituted in the following Equation 12 to figure out the target vascular resistance value. Constant H in Equation 12 is used to correct the non-linearity of vascular resistance. Adjustment of constant H helps to maintain the normal operation of the system even in a subject with a highly non-linear vascular resistance.

### Equation 12

$$\text{(target vascular resistance value)} = \{(\text{target arterial blood pressure value}) - (\text{target Pra value}) - H\} \big/ (\text{target CO value})$$

(where H is a constant)

The above-mentioned target values of CO, Pla, Pra, and AP are input to target decision means (7) by a user to obtain a desired patient condition.

Calculation means (3) carries out computation using the hemodynamic values input from input means (2). Calculation means (3) includes a first calculation means (31), a second calculation means (32), and a third calculation means (33). Calculation means (3) may be a single processing unit that plays roles of first to third calculation means (31-33) in performing computations. Alternatively, calculation means (3) may include three processing units that respectively play roles of first to third calculation means (31-33) in performing computation.

First calculation means (31) calculates a pumping ability value of the left heart from the CO value and the Pla value input from input means (2). Alternatively, first calculation means calculates a target pumping ability value of the right heart from the CO value and the Pra value input from input means (2). First calculation means (31) may be configured to calculate both or either of the above-mentioned pumping ability value of the left heart and/or the right heart.

In calculation of a pumping ability value of the left heart by first calculation means (31), the CO value and Pla value input from input means (2) are substituted in the following Equation 13 to figure out the pumping ability value of the left heart.

In Equation 13, constants A and B are user preset values that are adjustable for each patient. Adjustment of these values for each patient allows the calculated pumping ability value of the left heart to be corrected.

### Equation 13

$$\text{(CO value)} = \text{(pumping ability value of the left heart)} \times \{\text{Log}((\text{Pla value}) - A) + B\}$$

(where both A and B are constants)

In using the above equation, the equation is converted into an equation such as Equation 14 shown below to allow the pumping ability value of the left heart to be calculated from the input values sent from input means (2). Use of the converted equation enables fast arithmetic processing.

## Equation 14

$$\text{(pumping ability value of the left heart)} = \frac{\text{(CO value)}}{\{\text{Log}((\text{Pla value}) - A) + B\}}$$

(where both A and B are constants)

First calculation means (31) calculates a pumping ability value of the right heart using the following Equation 15 by a similar calculation to the above-described calculation of the pumping ability value of the left heart. The pumping ability value of the right heart is calculated by substituting the CO value and the Pra value in Equation 15.

First calculation means 31 is provided with Equation 16 converted from Equation 16 to allow the pumping ability value of the right heart to be calculated from the values input from input means (2).

In Equations 15 and 16, constants C and D are user preset values that are adjustable for each patient. Adjustment of these values for each patient allows the calculated pumping ability value of the right heart to be corrected.

## Equation 15

$$\text{(CO value)} = \text{(pumping ability value of the right heart)} \times \{\text{Log}((\text{Pra value}) - C) + D\}$$

(where both C and D are constants)

## Equation 16

$$\text{(pumping ability value of the right heart)} = \frac{\text{(CO value)}}{\{\text{Log}((\text{Pra value}) - C) + D\}}$$

(where both C and D are constants)

The pumping ability value of the left heart and the pumping ability value of the right heart are both calculated by first calculation means (31) using the CO value, Pla value and Pra value, it is possible to show the pumping ability value of the left heart and the pumping ability value of the right heart by plotting the relationship among CO value, Pla value and Pra value using three axes for these, as shown in Fig. 3.

By plotting the values of CO, Pla, and Pra on three axes of a three dimensional coordinate, the change in CO is represented as CO curve in the three dimensional coordinate. The CO curve visualizes the integrated pumping ability of the left heart and the right heart so as to significantly facilitate the diagnosis on a patient.

Fig. 3 shows CO curve (a) of a normal heart and CO curve (b) of a failing heart. One can easily read from CO curves (a) and (b) that the failing heart has a lower integrated pumping ability of the left heart and the right heart. The representation of a normal CO curve and a measured CO curve in one space facilitates the diagnosis.

Calculation means (3) includes second calculation means (32).

Second calculation means (32) calculates an effective circulating blood volume value from the CO value, the Pla value, and the Pra value.

In calculation of an effective circulating blood volume value by second calculation means (32), the CO value, the Pla value, and the Pra vale input from input means (2) are substituted in the following Equation 17 to figure out the effective circulating blood volume value.

In Equation 17, constants E, F and G are user preset values that are adjustable for each patient. Adjustment of these values for each patient allows the calculated effective circulating blood volume value to be corrected.

## Equation 17

$$\text{(CO value)} = \frac{1}{E} \text{(effective circulating blood volume value)} - F \text{(Pra value)} - G \text{(Pla value)}$$

(where all of E, F, and G are constants)

In using the above Equation 17, the equation is converted into an equation such as Equation 18 shown below to allow the effective circulating blood volume value to be calculated from the input values from input means (2). Use of the converted equation enables fast arithmetic processing.

## Equation 18

$$(\text{effective circulating blood volume value}) = \{(\text{CO value}) + F\,(\text{Pra value}) + G\,(\text{Pla value})\} \times E$$

(where all of E, F, and G are constants)

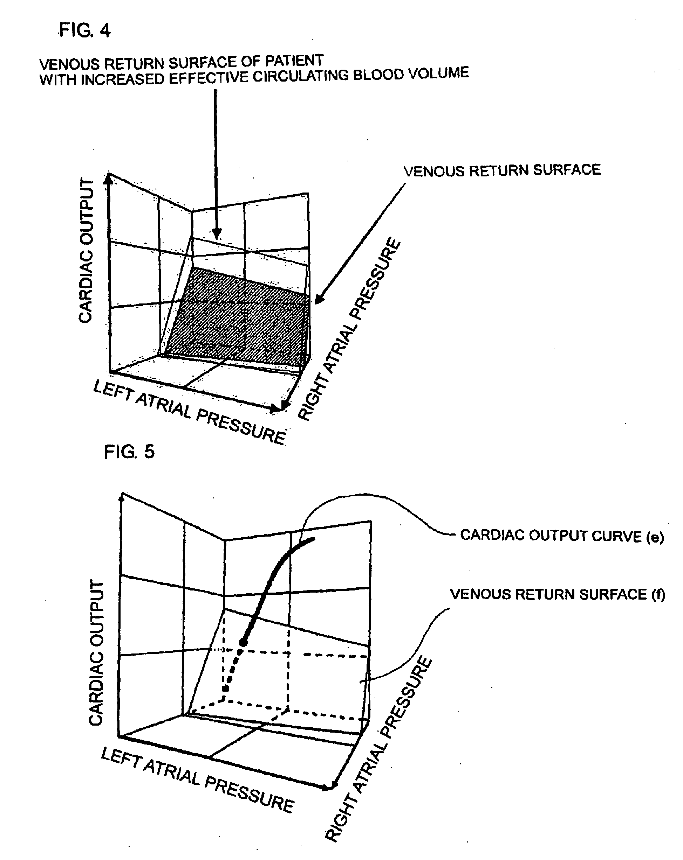

The CO value, the Pra value, and the Pla value used for calculation by second calculation means (32) can be plotted on three axes of a three dimensional coordinate. The CO value, the Pla value, and the Pra value as three variables form a plane (venous return surface) as shown in Fig. 4..

The venous return surface is defined by the three variables (CO value, Pla value, and Pra value) and constants E, F, and G. The slope of the surface may be considered to be constant between different subjects or within a single subject. It is also possible to correct the slope for a particular group of patients.

The height of the venous return surface is determined by the calculated effective circulating blood volume value, where the slope is considered to be constant.

By plotting the values of CO, Pla, and Pra on three axes of a three dimensional coordinate, the effective circulating blood volume is represented in the venous return surface. This visualizes the effective circulating blood volume so as to significantly facilitate the diagnosis of a patient.

Fig. 4 shows venous return surface (c) of a normal subject and venous return surface (d) of a congestive patient where the effective circulating blood volume is increased. Venous return surface (d) is upwardly shifted from venous return surface (c). The representation of a normal venous surface and a measured venous surface in one space facilitates the diagnosis.

Calculation means (3) includes third calculation means (33).

Third calculation means (33) calculates a vascular resistance value from the CO value, the Pra value, and the AP value.

In calculation of a vascular resistance value by third calculation means (33), the CO value, the Pra value, and the AP value input from input means (2) are substituted in the following Equation 19 to figure out the vascular resistance value.

Constant H in Equation 19 is used to correct the non-linearity of vascular resistance. Adjustment of constant H helps to maintain the normal operation of the system even in a subject with a highly non-linear vascular resistance.

## Equation 19

$$(\text{vascular resistance value}) = \frac{\{(\text{arterial blood pressure value}) - (\text{Pra value}) - H\}}{(\text{CO value})}$$

(where H is a constant)

Fig. 5 shows one embodiment of simultaneous representation of CO curve (e) and venous return surface (f) in a three dimensional coordinate diagram with three axes respectively plotting the CO value, the Pla value, and the Pra value.

In Fig. 5, the intersection point of CO curve (e) with venous return surface (f) gives the CO value, the Pla value, and the Pra value of the subject.

If the CO value, the Pla value, and the Pra value are abnormal and need improvement to a certain target value, a target point corresponding to the target value is defined in the three dimensional coordinate diagram. Until the target point coincides with the intersection point of CO curve (e) and venous return surface (f), the pumping abilities of the left heart and the right heart as represented in CO curve (e) and the effective circulating blood volume as represented in venous return surface are controlled by drug administration to the subject. This enables the CO value, the Pla value, and the Pra value to achieve the normal target values.

Comparison means (4) compares the values calculated in calculation means (3) and the target values of pumping ability (pumping ability of the left heart and/or the right heart), effective circulating blood volume, and vascular resistance calculated in target decision means (7). Comparison means (4) includes a first comparison means (41), a second comparison means (42), and a third comparison means (43).

First comparison means (41) compares the pumping ability value of the left heart and /or the pumping ability value of the right heart calculated in first calculation means (31) and the target pumping ability value of the left heart value and/or the target pumping ability value of the right heart calculated in first target decision means (71). More specifically, after first calculation means (31) calculates the pumping ability value of the left heart, first comparison means (41) compares the calculated pumping ability value of the left heart and the target pumping ability value of the left heart calculated in first target decision means (71), and then sends dosing means (5) a signal indicating the result of the comparison.

Through the comparison, first comparison means (41) finds out that the calculated pumping ability value of the left heart is larger than, equal to, or smaller than the target pumping ability value of the left heart. According to this comparison result, first comparison means (41) sends the dosing means (first dosing means (51)) described below a signal indicating that the calculated pumping ability value of the left heart is larger than (signal "large"), equal to (signal "equal"), or smaller than (signal "small") the target pumping ability value of the left heart. In addition to one of signal "large", signal "equal", and signal "small", first comparison means (41) may send the dosing means a signal indicating the quantified difference between the calculated pumping ability value of the left heart and the target pumping ability value of the left heart.

The target pumping ability value of the left heart may be set by a user as a default value, as described above. Although the example of the pumping ability value of the left heart has been described above, the pumping ability value of the right heart may be set in a similar way to the pumping ability value of the left heart. It is possible to use the pumping ability value of either the left heart or the right heart because they may exhibit an identical change (behavior).

Second comparison means (42) compares the effective circulating blood volume value calculated in second calculation means (32) and the target effective circulating blood volume value calculated in second target decision means (72). More specifically, after second calculation means (32) calculates the effective circulating blood volume value, second comparison means (42) compares the calculated effective circulating blood volume value and the target effective circulating blood volume value calculated in second target decision means (72), and then sends dosing means (5) a signal indicating the result of the comparison.

Through the comparison, second comparison means (42) finds out that the calculated effective circulating blood volume value is larger than, equal to, or smaller than the target effective circulating blood volume value. According to this comparison result, second comparison means (42) sends the dosing means (second dosing means (52)) described below a signal indicating that the calculated effective circulating blood volume value is larger than (signal "large"), equal to (signal "equal"), or smaller than (signal "small") the target effective circulating blood volume value. In addition to one of signal "large", signal "equal", and signal "small", second comparison means (42) may send the dosing means a signal indicating the quantified difference between the calculated effective circulating blood volume value and the target effective circulating blood volume value.

The target effective circulating blood volume value may be set by a user as a default value, as described above.

Third comparison means (43) compares the vascular resistance value calculated in third calculation means (33) and the target vascular resistance value calculated in third target decision means (73). More specifically, after third calculation means (33) calculates the vascular resistance value, third comparison means (43) compares the calculated vascular resistance value and the target vascular resistance value calculate in third target decision means (73), and then sends dosing means (5) a signal indicating the result of the comparison.

Through the comparison, third comparison means (43) finds out that the calculated vascular resistance value is larger than, equal to, or smaller than the target vascular resistance value. According to this comparison result, third comparison means (43) sends the dosing means (third dosing means (53)) described below a signal indicating that the calculated vascular resistance value is larger than (signal "large"), equal to (signal "equal"), or smaller than (signal "small") the target vascular resistance value. In addition to one of signal "large", signal "equal", and signal "small", third comparison means (43) may send the dosing means a signal indicating the quantified difference between the calculated vascular resistance value and the target vascular resistance value.

The target vascular resistance value may be set by a user as a default value, as described above.

Dosing means (5) receives the comparison results from comparison means (4) to start and/or stop drug administration to a patient according to the comparison results.

Dosing means (5) includes a first dosing means (51), a second dosing means (52), and a third dosing means (53). First to third dosing means (51-53) are configured to administer drugs to a patient, as described below, and to control the drug administration (adjust the amount of drugs to be administered) to a patient using the signals sent by comparison means (4) according to the comparison results.

Dosing means (5) may be, for example, a multilumen type catheter for simultaneous infusion of multiple drugs that is connected to multiple automatic infusion pumps. The catheter is placed in the vein of a patient for intravenous administration of drugs.

First dosing means (51) administers a certain drug into the body of the patient according to the signal of the comparison results sent from first comparison means (41).

The drug administration according to the signal of the comparison results from first comparison means (41) allows first dosing means (51) to start drug administration when abnormal pumping ability is detected.

For example, first dosing means (51) receives a signal "small" (a signal sent when the calculated pumping ability value of the left heart is smaller than the target pumping ability value) from first comparison means (41). Since the signal indicates abnormal pumping ability, first dosing means (51) starts drug administration to improve pumping ability Inotropic agent such as dobutamine and dopamine is administered in this drug administration.

In another case, first dosing means (51) receives a signal "equal" (a signal sent when the calculated pumping ability value of the left heart is equal to the target pumping ability value) from first comparison means (41). Since the signal indicates normal pumping ability, first dosing means (51) responds by not increasing the dose of the drug, by administering no drug, or by stopping administration of the drug.

In yet another case, first dosing means (51) receives a signal "large" (a signal sent when the calculated pumping ability value of the left heart is larger than the target pumping ability value) from first comparison means (41). Since the signal indicates better pumping ability than targeted, first dosing means (51) responds by reducing the dose of the inotropic agent, or by not administering the inotropic agent, or by stopping administration of the inotropic agent.

The dose of the drugs administered by first dosing means (51) is not particularly limited but an appropriate dose for a patient is primarily selected. It is possible to vary the dose based on the difference between the calculated pumping ability value and the target pumping ability value, which are compared by first comparison means (41). Although, in the above description, the operation of first dosing means (51) is controlled by the signals obtained by dividing calculated pumping ability values into three levels according to whether the calculated pumping ability value is larger than, equal to, smaller than the target pumping ability value, it is also possible to divide the calculated pumping ability values into multiple levels according to the deviation from the target pumping ability value and then to vary the dose of drugs. The variation of dose according to the multiple levels results in highly accurate administration of the drugs.

Second dosing means (52) administers a certain drug into the body of the patient according to the signal of the comparison results sent from second comparison means (42).

The drug administration according to the signal of the comparison results from second comparison means (42) allows second dosing means (52) to start drug administration when abnormal effective circulating blood volume is detected.

For example, second dosing means (52) receives a signal "large" (a signal sent when the calculated effective circulating blood volume value is larger than the target effective circulating blood volume value) from second comparison means (42). Since the signal indicates abnormal effective circulating blood volume, second dosing means (52) starts drug administration to reduce effective circulating blood volume. Diuretic such as furosemide is administered in this drug administration.

In another case, second dosing means (52) receives a signal "equal" (a signal sent when the calculated effective circulating blood volume value is equal to the target effective circulating blood volume value) from second comparison means (42). Since the signal indicates normal effective circulating blood volume, second dosing means (52) responds by administering no drug or by stopping administration of the drug.

In yet another case, second dosing means (52) receives a signal "small" (a signal sent when the calculated effective circulating blood volume value is smaller than the target effective circulating blood volume value) from second comparison means (42). Since the signal indicates abnormal effective circulating blood volume, second dosing means (52) starts drug administration to increase effective circulating blood volume. Infusion of a drug for increasing effective circulating blood volume such as low molecular dextran or albumin preparation is administered in this drug administration.

The dose of the drugs administered by second dosing means (52) is not particularly limited but an appropriate dose for a patient is primarily selected, as described for first dosing means (51). It is possible to vary the dose based on the difference between the calculated effective circulating blood volume value and the target effective circulating blood volume value, which are compared by second comparison means (42). Specifically, the calculated effective circulating blood volume values are divided into multiple levels according to the deviation from the target effective circulating blood volume value and then the dose of drugs is varied according to the levels. The variation of dose according to the multiple levels results in highly accurate administration of the drugs.

Third dosing means (53) administers a certain drug into the body of the patient according to the signal of the comparison results sent from third comparison means (43).

The drug administration according to the signal of the comparison results from third comparison means (43) allows third dosing means (53) to start drug administration when abnormal vascular resistance is detected.

For example, third dosing means (53) receives a signal "large" (a signal sent when the calculated vascular resistance value is larger than the target vascular resistance value) from third comparison means (43). Since the signal indicates abnormal vascular resistance, third dosing means (53) starts drug administration to reduce vascular resistance. Vasodilator such as nitroprusside, nitroglycerin, and phentolamine is administered in this drug administration. If vasoconstrictor such as norepinephrine has been already administered, the dose of the vasoconstrictor is reduced.

In another case, third dosing means (53) receives a signal "equal" (a signal sent when the calculated vascular resistance value is equal to the target vascular resistance value) from third comparison means (43). Since the signal indicates normal vascular resistance, third dosing means (53) responds by administering no drug or by stopping administration of the drug.

In yet another case, third dosing means (53) receives a signal "small" (a signal sent when the calculated vascular resistance value is smaller than the target vascular resistance value) from third comparison means (43). Since the signal indicates abnormal vascular resistance value, third dosing means (53) starts drug administration to increase vascular resistance. Vasoconstrictor such as norepinephrine is administered in this drug administration. If vasodilator such as nitroprusside, nitroglycerin, and phentolamine has been already administered, the dose of the vasodilator is reduced.

The dose of the drugs administered by third dosing means (53) is not particularly limited but an appropriate dose for a patient is primarily selected, as described for the first dosing means (51). It is possible to vary the dose based on the difference between the calculated vascular resistance value and the target vascular resistance value, which are compared by third comparison means (43). Specifically, the calculated vascular resistance values are divided into multiple levels according to the deviation from the target vascular resistance value and then the dose of drugs is varied according to the levels. The variation of dose according to the multiple levels results in highly accurate administration of the drugs.

To control the current value of pumping ability, effective circulating blood volume, and vascular resistance, first to third dosing means (51-53) may be configured to use time-based control to achieve the target values of pumping ability, effective circulating blood volume, and vascular resistance, which are determined by first to third comparison means (41-43). PI (Proportional Integral) control or PID (Propotional Integral Derivative) control is preferably used in such control, although other control laws may be applicable.

The PI control and PID control on the dose of drugs results in highly accurate administration.

For patients who manifest an idiosyncratic response to drugs, adaptive control is used in adjusting the dose of drugs.

Display means (6) is an output device that displays the input values of CO, Pla, Pra, and AP input by input means (2) as well as the calculated values obtained by the above-described first to third calculation means (31-33).

Display means (6) displays the calculated values of pumping ability, effective circulating blood volume, and vascular resistance, which are calculated in each calculation means (31-33), preferably in a continuous time-series line plot because each calculation means (31-33) continuously obtains calculated values from the input values obtained in the above-described continuous measurement of patient conditions by cardiac disease treatment system (1).

The continuous time-series line plot visualizes a time-series change and makes the change easily noticed.

Display means (6) is preferably configured to display the dose of drugs administered by first to third dosing means (51-53). The additional display of the drug dose effectively allows a user to observe the change (transition) in pumping ability, effective circulating blood volume, and vascular resistance of a patient simultaneously with the dose of drugs.

The configurations of cardiac disease treatment system (1) according to the present invention have been described thus far.

The operation of Cardiac disease treatment system (1) according to the present invention will be described below.

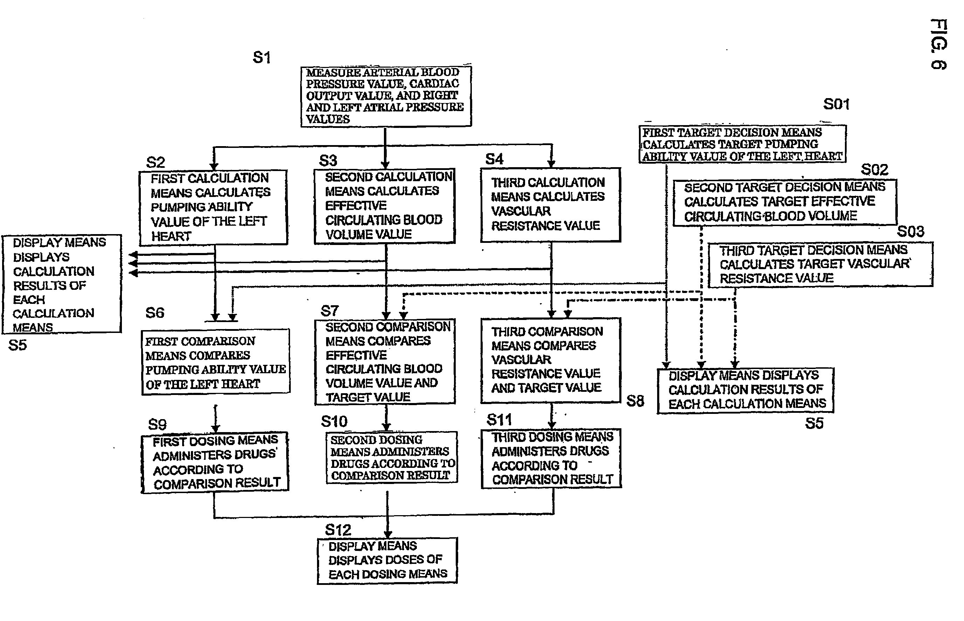

Fig. 6 is a flow chart illustrating the use of the cardiac disease treatment system according to the present invention.

Although cardiac disease treatment system (1) needs preset constants A to H in a practical use, description of these constants is omitted here. Target values of CO, Pra, Pla, and AP used by first to third target decision means (71-73) of target decision means (7) are set by a user to a certain value in the following example.

First, a user inputs target values of CO, Pra, Pla, and AP which represent desired patient conditions.

From the target value of CO, Pra, Pla, and AP, target decision means (7) calculates target values of pumping ability of the left heart, pumping ability of the right heart, effective circulating blood value, and vascular resistance.

Specifically, once the target CO value and the target Pla value are input to first decision means (71), it calculates a target pumping ability value of the left heart. In addition, once the target CO value and the target Pra value are input to first target decision means (71), it calculates a target pumping ability value of the right heart (SO1).

Once the target CO value, the target Pla value, and the target Pra value are input to second target decision means (72), it calculates a target affective circulating blood volume value (S02).

Once the target CO value, the target Pra value, and the target AP value are input to third target decision means (73), it calculates a target vascular resistance value (S03).

After respective target values of pumping ability of the left heart, pumping ability of the right heart, effective circulating blood volume, and vascular resistance are calculated by target decision means (7), these target values are sent to display means (6). The target values sent to display means (6) are respectively displayed in continuous time-series line plot (see S5 described below).

Then AP, CO, Pra, and Pla of a patient are measured (S1).

A Swan-Ganz catheter is connected to a patient in this measurement, although other apparatus may be used.

The measurement values of AP, CO, Pra, and Pla are input by input means (2) to calculation means (3).

Once these measurement values are input by input means (2), calculation means (3) calculates a pumping ability value of the left heart, a pumping ability value of the right heart, an effective circulating blood volume value, and a vascular resistance value. Specifically, once the CO value and the Pla value are input to first calculation means, it calculates as pumping ability value of the left heart. In addition, once CO value and Pra value are input to first calculation means (31), it calculates a pumping ability value of the right heart (S2).

Once the CO value, the Pla value, and the Pra value are input to second calculation means (32), it calculates an effective circulating blood volume value (S3).

Once the CO value, the Pra value, and the AP value are input to third calculation means (33), it calculates a vascular resistance value (S4).

From each measurement value of CO, Pla, Pra, and AP, first to third calculation means (31-33) respectively calculate a pumping ability value of the left heart, a pumping ability value of the right heart, an effective circulating blood volume value, and a vascular resistance value.

After respective calculated values of pumping ability of the left heart, pumping ability of the right heart, effective circulating blood volume, and vascular resistance are obtained by calculation means (3), these calculated values are sent to display means (6). The calculated values sent to display means (6) are respectively displayed in continuous time-series line plot (see S5 described below).

Then the pumping ability value of the left heart calculated in first calculation means (31) is sent to first comparison means (41). On the other hand, first comparison means (41) receives the target pumping ability value of the left heart calculated in first target decision means (71). First comparison means (41) compares the pumping ability value of the left heart (or the pumping ability value of the right heart) with the target pumping ability value of the left heart (or the target pumping ability value of the right heart) (S6).

First comparison means (41) sends first dosing means (51) the results of comparison between the calculated pumping ability value of the left heart and the target pumping ability value of the left heart.

The effective circulating blood volume value calculated in second calculation means (32) is sent to second comparison means (42). On the other hand, second comparison means (42) receives the target effective circulating blood volume value calculated in second target decision means (72). Second comparison means (42) compares the effective circulating blood volume value with the target effective circulating blood volume value (S7).

Second comparison means (42) sends second dosing means (52) the results of comparison between the calculated effective circulating blood volume value and the target effective circulating blood volume value.

The vascular resistance value calculated in third calculation means (33) is sent to third comparison means (43). On the other hand, third comparison means (43) receives the target vascular resistance value calculated in third target decision means (73). Third comparison means (43) compares the vascular resistance value and the target vascular resistance value (S8).

Third comparison means (43) sends third dosing means (53) the results of comparison between the calculated vascular resistance value and the target vascular resistance value.

First dosing means (51) receives the comparison result obtained in first comparison means (41) and then administers drugs according to this comparison result (S9).

If the comparison result from first comparison means (41) shows that first dosing means (51) should promote drug administration (if the calculated pumping ability value is smaller than the target pumping ability value), first dosing means (51) administers inotropic agent to a patient to increase the pumping ability value of the patient.

If the comparison result from first comparison means (41) shows that first dosing means (51) should stop drug administration or should administer no drug (if the calculated pumping ability value is equal to the target pumping ability value), first dosing means (51) stops drug administration or continues with no drug administration.

If the comparison result from first comparison means (41) shows that first dosing means (51) should administer no drug (if the calculated pumping ability value is larger than the target pumping ability value), first dosing means (51) reduces the dose of inotropic agent, or stops administration of inotropic agent, or continues with no administration of inotropic agent.

Second dosing means (52) receives the comparison result obtained in second comparison means (42) and then administers drugs according to this comparison result (S10).

If the comparison result from second comparison means (42) shows that second dosing means (51) should promote drug administration (if the calculated effective circulating blood volume value is smaller than the target effective circulating blood volume value), second dosing means (52) responds by administering an appropriate infusion of drugs such as low molecular dextran and albumin preparation to a patient to increase the effective circulating blood volume value of the patient.

If the comparison result from second comparison means (42) shows that second dosing means (52) should stop drug administration or should administer no drug (if the calculated effective circulating blood volume value is equal to the target effective circulating blood volume value), second dosing means (52) stops drug administration or continues with no drug administration.

If the comparison result from second comparison means (42) shows that second dosing means (52) should promote drug administration (if the calculated effective circulating blood volume value is larger than the target effective circulating blood volume value), second dosing means (52) administers diuretic to a patient to reduce effective circulating blood volume of the patient.

Third dosing means (53) receives the comparison result obtained in third comparison means (43) and then administers drugs according to this comparison result (S11).

If the comparison result from third comparison means (43) shows that third dosing means (53) should promote drug administration (if the calculated vascular resistance value is smaller than the target vascular resistance value), third dosing means (53) administers vasoconstrictor to a patient to increase the vascular resistance value of the patient.

If the comparison result from third comparison means (43) shows that third dosing means (53) should stop drug administration or should administer no drug (if the calculated vascular resistance value is equal to the target vascular resistance value), third dosing means (53) stops drug administration or continues with no drug administration.

If the comparison result from third comparison means (43) shows that third dosing means (53) should promote drug administration (if the calculated vascular resistance value is larger than the target vascular resistance value), third dosing means (53) administers vasodilator to a patient to reduce the vascular resistance of the patient.

As described above, first to third dosing means (51-53) may use a multi-level control or a real time control (PI control, PID control, or the like) on the dose. First to third dosing means (51-53) may use an adaptive control on the dose for patients who manifest an idiosyncratic response to drugs.

Display means (6) displays the time sequence of the pumping ability values (pumping ability value of the left heart and pumping ability value of the right heart), the effective circulating blood volume value, and the vascular resistance value, each calculated by first to third calculation means (31-33); the target values of pumping ability (pumping ability of the left heart and pumping ability of the right heart), effective circulating blood volume, and vascular resistance, each calculated by first to third target decision means (71-73); and the dose of drugs administered according to these calculated values (S12).

The display of these calculated values, target values, and the dose of drugs by display means (6) allows a user of cardiac disease treatment system (1) to carry out real time diagnosis on patient conditions.

## EXAMPLE 1

The results in the tests on cardiac disease treatment system (1) according to the present invention will be shown below.

In this example, the accuracy of the equations (the above-described equations 13 to 19) used by cardiac disease treatment system (1) according to the present invention was tested.

Seven dogs were used to examine the effectiveness of these equations.

First, constants A and B in equations 13 and 14 are examined. These constants A and B are employed in calculating the pumping ability of the left heart of the dogs.

As shown in the below table 1, constants A and B for dogs may be determined as A=2.03 and B=0.80.

Table 1

| Dog | pumping ability of the left heart | A | B | Coefficient of determination | Standard error of estimate |
|---|---|---|---|---|---|
| 1 | 58.1 | 1.27 | 0.61 | 0.98 | 4.3 |
| 2 | 24.4 | 2.03 | 2.71 | 0.95 | 3.6 |
| 3 | 108.4 | 0.00 | -0.67 | 0.95 | 5.6 |
| 4 | 66.7 | 2.08 | 0.08 | 0.98 | 5.9 |
| 5 | 105.6 | 2.30 | -0.02 | 0.99 | 5.0 |
| 6 | 73.5 | 2.21 | 0.59 | 0.99 | 2.5 |
| 7 | 42.0 | 4.32 | 2.30 | 0.98 | 4.7 |
| Average | 68.4 | 2.03 | 0.80 | 0.97 | 4.5 |
| Standard Deviation | 30.9 | 1.29 | 1.25 | | 1.2 |

Next, constants C and D in equations 15 and 16 are examined. These constants C and D are employed in calculating the pumping ability of the right heart of the dogs.

As shown in the below table 2, constants C and D for dogs may be determined as C=1.0 and D=0.88.

Constants C and D are obtained by linear regression to the measurement values listed in table 2 and a modification for the practical use.

Table 2

| Dog | Pumping ability of the right heart | C | D | Coefficient of determination | Standard error of estimate |
|---|---|---|---|---|---|
| 1 | 46.7 | 2.12 | 2.34 | 0.98 | 4.7 |
| 2 | 33.9 | 1.50 | 2.50 | 0.96 | 3.3 |
| 3 | 64.1 | 2.10 | 2.10 | 0.90 | 8.2 |
| 4 | 112.7 | 1.39 | 0.19 | 0.98 | 5.5 |
| 5 | 101.8 | 1.39 | 0.92 | 0.99 | 4.6 |
| 6 | 80.6 | 3.07 | 1.59 | 0.99 | 2.8 |
| 7 | 37.1 | 3.33 | 3.69 | 0.94 | 6.8 |
| Average | 68.1 | 2.13 | 1.90 | 0.96 | 5.1 |
| Standard Deviation | 31.3 | 0.80 | 1.14 | | 1.9 |

Next, constants E, F, and G in equations 17 and 18 are examined. These constants E, F, and G are employed in calculating the effective circulating blood volume of the dogs.

As shown in the below table 3, constants E, F, and G for dogs may be determined as E=0.129, F=19.61, and G=3.49.

Table 3

| Dog | E | F | G | Coefficient of determination | Standard error of estimate |
|---|---|---|---|---|---|
| 1 | 0.154 | 19.77 | 3.83 | 0.99 | 1.9 |
| 2 | 0.232 | 23.30 | 3.39 | 0.92 | 7.4 |
| 3 | 0.099 | 20.99 | 4.04 | 0.97 | 3.7 |
| 4 | 0.088 | 17.60 | 2.92 | 0.99 | 1.3 |
| 5 | 0.092 | 16.17 | 3.46 | 0.92 | 6.5 |
| 6 | 0.107 | 19.01 | 4.26 | 0.99 | 1.5 |
| 7 | 0.132 | 20.44 | 2.55 | 0.99 | 1.2 |
| Average | 0.129 | 19.61 | 3.49 | 0.97 | 3.4 |
| Standard Deviation | 0.051 | 2.33 | 0.61 | | 2.6 |

In this way, the default values of constants A to G are determined. Specifically, if the subjects (patients) are dogs, constants A to G are determined as A=2.03, B=0.80, C=1.0, D=0.88, E=0.129, F=19.61, and G=3.49. Constant H in Equation 19 was determined as H=0 for dogs, according to the study by Shoukas et. al. (see, for example, Shoukas AA. "Carotid sinus baroreceptor reflex control and epinephrine. Influence on capacitive and resistive properties of the total pulmonary vascular bed of the dog." Circ Res 51:95-101, 1982).

Using these constants, cardiac disease treatment system (1) in the present invention was operated.

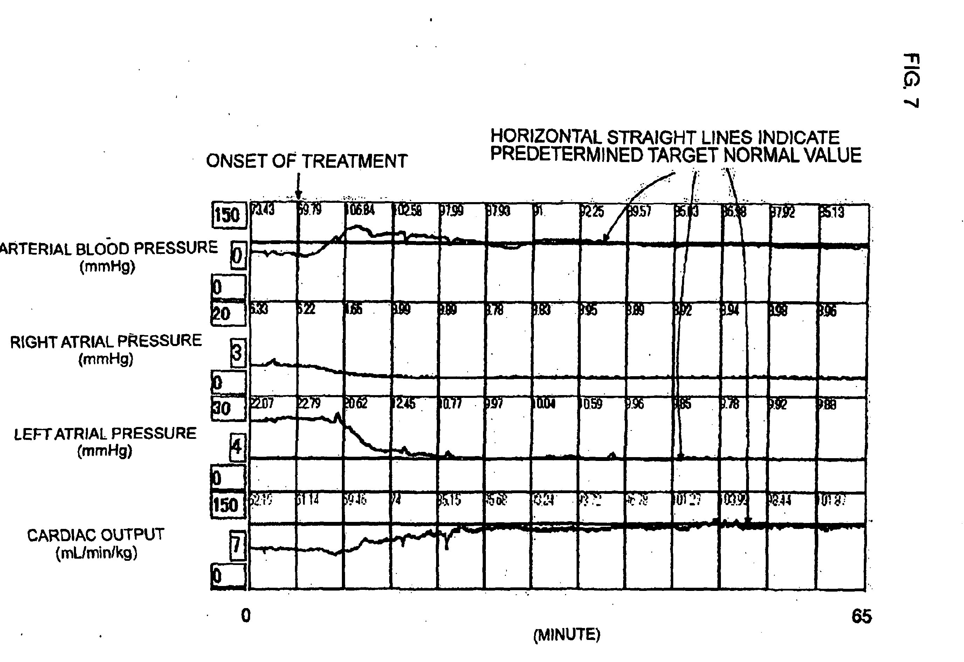

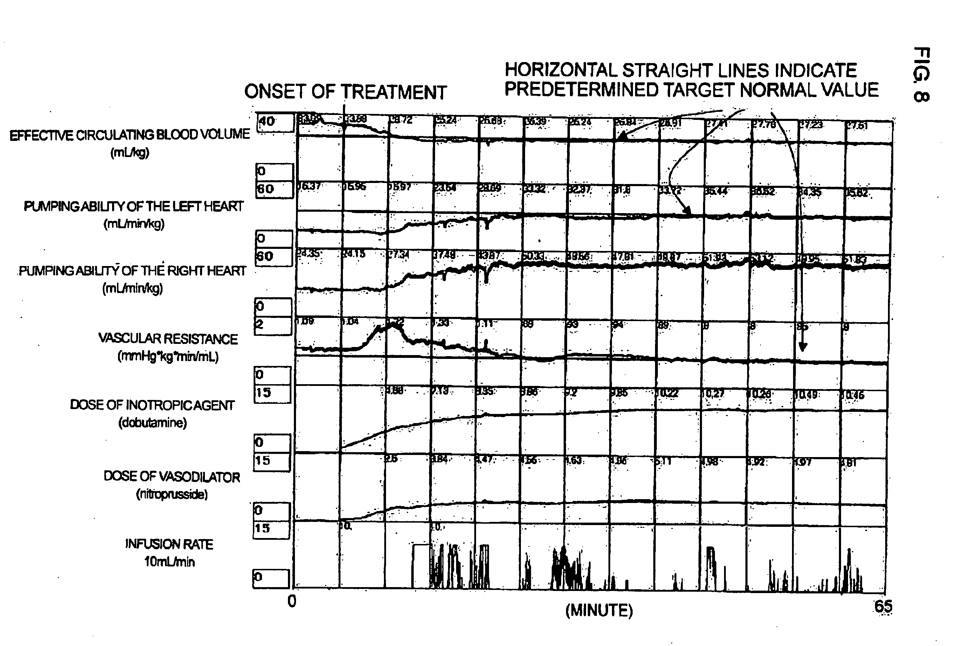

Figs. 7 and 8 show one example of the use of the cardiac disease treatment system according to the present invention.

Fig. 7 shows the chronological change in AP, right atrial pressure (Pra), left atrial pressure (Pla), and cardiac output (CO). As shown in Fig. 7, AP, Pra, Pla, and CO achieve target values within 20 minutes from the onset of treatment. The target values in this example were set as arterial blood pressure value=90mmHg, Pla value=10mmHg, and CO value=100ml/min/kg.

Fig. 8 shows a chronological change in effective circulating blood volume, pumping ability of the left heart, pumping ability of the right heart, vascular resistance, the dose of inotropic agent and vasodilator, and infusion amount. As inotropic agent and vasodilator, dobutamine and nitroprusside are used respectively. Low molecular dextran infusion is used. PI control is used in determining the dose of inotropic agent and vasodilator while multi-level control is used in determining the infusion amount.

At the onset of treatment, the effective circulating blood volume value is in excess of the target effective circulating blood volume value; the pumping ability value of the left heart and the pumping ability value of the right heart are lower than the target pumping ability value of the left heart and the target pumping ability value of the right heart; and the vascular resistance value is higher than the target vascular resistance value, as shown in Fig. 8. All of these values are significantly different from the target values to be in an abnormal range (calculation means (3) calculates each of the effective circulating blood volume value, the left pumping ability value of the left heart, the pumping ability value of the right heart, and the vascular resistance value. Comparison means (4) determines the condition of these parameters (whether they are in a normal or abnormal range)).

Once the treatment is started, dosing means (5) starts administering drugs to allow each calculated value to achieve each target value, according to the conditions (dosing means (5) administers an appropriate dose of drugs). The above-described processes are repeated until the respective calculated values achieve the target values.

As described above, it is possible to control the pumping ability value and the effective circulating blood volume value, and the vascular resistance value to desired values and thereby control the CO value, the Pla value, and the AP value to target values.

Description of the Numerals

1    Cardiac Disease Treatment System
2    Input Means
31   First Calculation Means
32   Second Calculation Means
33   Third Calculation Means
41   First Comparison Means
42   Second Comparison Means
43   Third Comparison Means
51   First Dosing Means
52   Second Dosing Means
53   Third Dosing Means
6    Display Means
7    Target Decision Means
71   First Target Decision Means
72   Second Target Decision Means
73   Third Target Decision Means

**Claims**

1.  A cardiac disease treatment system comprising:

    an input means (2) inputting a cardiac output value, a left atrial pressure value, a right atrial pressure value, and arterial blood pressure value of a patient;
    a first calculation means (31) calculating a pumping ability value of the left heart and or a pumping ability value of the right heart from the cardiac output value, the left atrial pressure value, and the right atrial pressure value which are input from the input means (2);
    a first comparison means (41) comparing calculated values of the pumping ability value of the left heart and/or the pumping ability value of the right heart and target pumping ability values; and
    a first dosing device (51) configured for administering drugs to the patient according to comparison results in the first comparison means (41);
    a second calculation means (32) calculating an effective circulating blood volume value from the cardiac output value, the left atrial pressure value, and the right atrial pressure value which are input from the input means (2);
    a second comparison means (42) comparing the effective circulating blood volume value calculated by the second calculation means (32) and a target effective circulating blood volume value;
    a second dosing device (52) configured for administering drugs to the patient according to the comparison result in the second comparison means (42);
    a third calculation means (33) calculating a vascular resistance value from the cardiac output value, the right atrial pressure value, and the arterial blood pressure value which are input by the input means (2);
    a third comparison means (43) comparing the vascular resistance value calculated by the third calculation means (33) and a target vascular resistance value; and
    a third dosing device (53) configured for administering drugs to the patient according to the comparison result in the third comparison means (43).

2. The cardiac disease treatment system according to claim 1, wherein the first calculation means (31) uses equation 1 and/or equation 2 to calculate the pumping ability value of the left heart and/or the pumping ability value of the right heart from the cardiac output value, the left atrial pressure value, and/or the right atrial pressure value input by the input means (2);

wherein the second calculation means (32) uses equation 5 to calculate the effective circulating blood volume value from the cardiac output value, the left atrial pressure value, and the right atrial pressure value input by the input means (2); and

wherein the third calculation means (33) uses equation 7 to calculate the vascular resistance value from the cardiac output value, the right atrial pressure value, and the arterial blood pressure value input by the input means (2).

### Equation 1

(cardiac output value)

= (pumping ability value of the left heart) x {Log ((left atrial pressure value) - A) + B}

wherein A and B are constants;

### Equation 2

(cardiac output value)

= (pumping ability value of the right heart) x {Log ((right atrial pressure value) - C) + D}

wherein C and D are constants;

### Equation 5

$$\text{(cardiac output value)} = \frac{1}{E} \text{(effective circulating blood volume value)}$$
$$- F \text{ (right atrial pressure value)} - G \text{ (left atrial pressure value)}$$

- F (right atrial pressure value) - G (left atrial pressure value)
wherein E, F, and G are constants;

### Equation 7

$$\text{(vascular resistance value)}$$
$$= \left\{\text{(arterial blood pressure value)} - \text{(right atrial pressure value)} - H\right\} \Big/ \text{(cardiac output value)}$$

wherein H is a constant.

3. The cardiac disease treatment system according to claim 1 or claim 2, further comprising:

a first target decision means (71) calculating a target pumping ability value of the left heart and/or a target pumping ability value of the right heart from a target cardiac output value, a target left atrial pressure value, and/or a target right atrial pressure value;

a second target decision means (72) calculating a target effective circulating blood volume value from a target cardiac output value, a target left atrial pressure value, and a target right atrial pressure value; and

a third target decision means (73) calculating a target vascular resistance value from a target cardiac output value, a target right atrial pressure value, and a target arterial blood pressure value.

4. The cardiac disease treatment system according to claim 3, wherein the first target decision means (71) uses equation 3 and equation 4 to calculate the target pumping ability value of the left heart and/or the target pumping ability value of the right heart from the target cardiac output value, the target left atrial pressure value, and/or the target right atrial pressure value;

wherein the second target decision means (72) uses equation 6 to calculate the target effective circulating blood volume value from the target cardiac output value, the target left atrial pressure value, and the target right atrial pressure value; and

wherein the third target decision means (73) uses equation 8 to calculate the target vascular resistance value from the target cardiac output value, the target right atrial pressure value, and the target arterial blood pressure value:

### Equation 3

$$(\text{target pumping ability value of the left heart})$$
$$= (\text{target cardiac output value}) \Big/ \{\text{Log}((\text{target left atrial pressure value}) - A) + B\}$$

wherein A and B are constants;

### Equation 4

$$(\text{target pumping ability value of the right heart})$$
$$= (\text{target cardiac output value}) \Big/ \{\text{Log}((\text{target right atrial pressure value}) - C) + D\}$$

wherein C and D are constants;

### Equation 6

$$(\text{target effective circulating blood volume value})$$
$$= \{(\text{target cardiac output value}) + F(\text{target right atrial pressure value}) + G(\text{target left atrial pressure value})\} \times E$$

whe **rein E, F, and G are constants;**

### Equation 8

$$(\text{target vascular resistance value})$$
$$= \{(\text{target arterial blood pressure value}) - (\text{target right atrial pressure value}) - H\} \Big/ (\text{target cardiac output value})$$

herein H is a constant.

5. The cardiac disease treatment system according to any one of claims 1 to 4, further comprising:

a display means (6) continuously displaying each of the calculated values calculated by the calculation means (31), (32), and (33) in chronological order.

6. The cardiac disease treatment system according to any one of claims 1 to 5, wherein the cardiac output value is measured by a Swan-Ganz catheter or calculated from a diastolic time constant of arterial blood pressure waveform.

7. The cardiac disease treatment system according to any one of claims 1 to 6, wherein the left atrial pressure value is directly measured by a catheter or continuously estimated from diastolic pressure values of pulmonary capillary wedge pressure or pulmonary arterial pressure which are measured by a Swan-Ganz catheter.

**Patentansprüche**

1. System zur Behandlung von Herzkrankheiten, umfassend:

   eine Eingabeeinrichtung (2) zur Eingabe eines Werts für das Herzzeitvolumen, eines Werts für den linksatrialen Druck, eines Werts für den rechtsatrialen Druck und eines Werts für den arteriellen Blutdruck eines Patienten;
   eine erste Berechnungseinrichtung (31) zum Berechnen eines Werts für die Pumpfähigkeit des linken Herzens und/oder eines Werts für die Pumpfähigkeit des rechten Herzens aus dem Wert für das Herzzeitvolumen, dem Wert für den linksatrialen Druck und dem Wert für den rechtsatrialen Druck, die über die Eingabeeinrichtung (2) eingegeben werden;
   eine erste Vergleichseinrichtung (41) zum Vergleichen der berechneten Werte des Werts für die Pumpfähigkeit des linken Herzens und/oder des Werts für die Pumpfähigkeit des rechten Herzens und der Zielwerte für die Pumpfähigkeit; und
   eine erste Dosiervorrichtung (51), die dazu ausgelegt ist, dem Patienten Arzneimittel gemäß den Vergleichsergebnissen der ersten Vergleichseinrichtung (41) zu verabreichen;
   eine zweite Berechnungseinrichtung (32) zum Berechnen eines Werts für das effektiv zirkulierende Blutvolumen aus dem Wert für das Herzzeitvolumen, dem Wert für den linksatrialen Druck und dem Wert für den rechtsatrialen Druck, die über die Eingabeeinrichtung (2) eingegeben werden;
   eine zweite Vergleichseinrichtung (42) zum Vergleichen des von der zweiten Berechnungseinrichtung (32) berechneten Werts für das effektiv zirkulierende Blutvolumen und eines Zielwerts für das effektiv zirkulierende Blutvolumen;
   eine zweite Dosiereinrichtung (52), die dazu ausgelegt ist, dem Patienten Arzneimittel gemäß dem Vergleichsergebnis der zweiten Vergleichseinrichtung (42) zu verabreichen;
   eine dritte Berechnungseinrichtung (33) zum Berechnen eines Werts für den vaskulären Widerstand aus dem Wert für das Herzzeitvolumen, dem Wert für den rechtsatrialen Druck und dem Wert für den arteriellen Blutdruck, die über die Eingabeeinrichtung (2) eingegeben werden;
   eine dritte Vergleichseinrichtung (43) zum Vergleichen des von der dritten Berechnungseinrichtung (33) berechneten Werts für den vaskulären Widerstand und eines Zielwerts für den vaskulären Widerstand; und
   eine dritte Dosiervorrichtung (53), die dazu ausgelegt ist, dem Patienten Arzneimittel gemäß dem Vergleichsergebnis der dritten Vergleichseinrichtung (43) zu verabreichen.

2. System zur Behandlung von Herzkrankheiten nach Anspruch 1, wobei die erste Berechnungseinrichtung (31) Gleichung 1 und/oder Gleichung 2 verwendet, um den Wert für die Pumpfähigkeit des linken Herzens und/oder den Wert für die Pumpfähigkeit des rechten Herzens aus dem über die Eingabeeinrichtung (2) eingegebenen Wert für das Herzzeitvolumen, dem Wert für den linskatrialen Druck und/oder dem Wert für den rechtsatrialen Druck zu berechnen;
   wobei die zweite Berechnungseinrichtung (32) Gleichung 5 verwendet, um den Wert für das effektiv zirkulierende Blutvolumen aus dem über die Eingabeeinrichtung (2) eingegebenen Wert für das Herzzeitvolumen, dem Wert für den linksatrialen Druck und dem Wert für den rechtsatrialen Druck zu berechnen; und
   wobei die dritte Berechnungseinrichtung (33) Gleichung 7 verwendet, um den Wert für den vaskulären Widerstand aus dem über die Eingabeeinrichtung (2) eingegebenen Wert für das Herzzeitvolumen, dem Wert für den rechtsatrialen Druck und dem Wert für den arteriellen Blutdruck zu berechnen.

## Gleichung 1

(Herzzeitvolumenwert)

= (Pumpfähigkeitswert des linken Herzens) x {Log((Wert für linksatrialen Druck) - A) + B}

wobei A und B Konstanten sind;

## Gleichung 2

(Herzzeitvolumenwert)

= (Pumpfähigkeitswert des rechten Herzens) x {Log((Wert für rechtsatrialen Druck) - C) + D}

wobei C und D Konstanten sind;

## Gleichung 5

$$(\text{Herzzeitvolumenwert}) = \frac{1}{E} (\text{Wert für effektiv zirkulierendes Blutvolumen})$$

$$- F (\text{Werf für rechtsatrialen Druck}) - G (\text{Wert für linksatrialen Druck})$$

wobei E, F und G Konstanten sind;

## Gleichung 7

(Wert für vaskulären Widerstand)

$$= \{(\text{Wert für arteriellen Blutdruck}) - (\text{Wert für rechtsatrialen Druck}) - H\} \Big/ (\text{Herzzeitvolumenwert})$$

wobei H eine Konstante ist.

**3.** System zur Behandlung von Herzkrankheiten nach Anspruch 1 oder Anspruch 2, ferner umfassend:

eine erste Zielentscheidungseinrichtung (71) zur Berechnung eines Zielwerts für die Pumpfähigkeit des linken Herzens und/oder eines Zielwerts für die Pumpfähigkeit des rechten Herzens aus einem Zielwert für das Herzzeitvolumen, einem Zielwert für den linksatrialen Druck und/oder einem Zielwert für den rechtsatrialen Druck; eine zweite Zielentscheidungseinrichtung (72) zur Berechnung eines Zielwerts für das effektiv zirkulierende Blutvolumen aus einem Zielwert für das Herzzeitvolumen, einem Zielwert für den linksatrialen Druck und einem Zielwert für den rechtsatrialen Druck; und eine dritte Zielentscheidungseinrichtung (73) zur Berechnung eines Zielwerts für den vaskulären Widerstand aus einem Zielwert für das Herzzeitvolumen, einem Zielwert für den rechtsatrialen Druck und einem Zielwert für den arteriellen Blutdruck.

**4.** System zur Behandlung von Herzkrankheiten nach Anspruch 3, wobei die erste Zielentscheidungseinrichtung (71) Gleichung 3 und Gleichung 4 verwendet, um den Zielwert für die Pumpfähigkeit des linken Herzens und/oder den Zielwert für die Pumpfähigkeit des rechten Herzens aus dem Zielwert für das Herzzeitvolumen, dem Zielwert für den linksatrialen Druck und/oder dem Zielwert für den rechtsatrialen Druck zu berechnen; wobei die zweite Zielentscheidungseinrichtung (72) Gleichung 6 verwendet, um den Zielwert für das effektiv zirkulierende Blutvolumen aus dem Zielwert für das Herzzeitvolumen, dem Zielwert für den linksatrialen Druck und dem Zielwert für den rechtsatrialen Druck zu berechnen; und wobei die dritte Zieleintscheidungseinrichtung (73) Gleichung 8 verwendet, um den Zielwert für den vaskulären Widerstand aus dem Zielwert für das Herzzeitvolumen, dem Zielwert für den rechtsatrialen Druck und dem Zielwert für den arteriellen Blutdruck zu berechnen:

## Gleichung 3

(Zielwert für Pumpfähigkeit des linken Herzens)

$$= (\text{Zielwert für Herzzeitvolumen}) \Big/ \{\text{Log}((\text{Zielwert für linksatrialen Druck}) - A) + B\}$$

wobei A und B Konstanten sind;

Gleichung 4

(Zielwert für Pumpfähigkeit des rechten Herzens)

= (Zielwert für Herzzeitvolumen) $\Big/$ {Log((Zielwert für rechtsatrialen Druck) − C) + D}

wobei C und D Konstanten sind;

Gleichung 6

(Zielwert für effektiv zirkulierendes Blutvolumen)

= {(Zielwert für Herzzeitvolumen) + F(Zielwert für rechtsatrialen Druck) + G(Zielwert für linksatrialen Druck))} x E

wobei E, F und G Konstanten sind;

Gleichung 8

(Zielwert für vaskulären Widerstand)

= {(Zielwert für arteriellen Blutdruck) − (Zielwert für rechtsatrialen Druck) − H} $\Big/$ (Zielwert für Herzzeitvolumen)

wobei H eine Konstante ist.

**5.** System zur Behandlung von Herzkrankheiten nach einem der Ansprüche 1 bis 4, ferner umfassend:

    eine Anzeigeeinrichtung (6) zur kontinuierlichen Anzeige jedes der durch die Berechnungseinrichtung (31), (32) und (33) berechneten Werte in chronologischer Reihenfolge.

**6.** System zur Behandlung von Herzkrankheiten nach einem der Ansprüche 1 bis 5, wobei der Wert für das Herzzeitvolumen mit einem Swan-Ganz-Katheter gemessen wird oder aus einer diastolischen Zeitkonstante der Wellenform des arteriellen Blutdrucks berechnet wird.

**7.** System zur Behandlung von Herzkrankheiten nach einem der Ansprüche 1 bis 6, wobei der Wert für den linksatrialen Druck direkt mit einem Katheter gemessen wird oder kontinuierlich aus den mit einem Swan-Ganz-Katheter gemessenen diastolischen Druckwerten des Drucks der Lungenkapillarweiche oder des pulmonalarteriellen Drucks geschätzt wird.

**Revendications**

**1.** Système de traitement d'une maladie cardiaque, comprenant :

    un moyen d'entrée (2) pour l'entrée d'une valeur de débit cardiaque, d'une valeur de pression auriculaire gauche, d'une valeur de pression auriculaire droite et d'une valeur de tension artérielle d'un patient ;
    un premier moyen de calcul (31) calculant une valeur de capacité de pompage du coeur gauche et/ou une valeur de capacité de pompage du coeur droit à partir de la valeur de débit cardiaque, de la valeur de pression auriculaire gauche et de la valeur de pression auriculaire droite qui sont entrées à partir du moyen d'entrée (2) ;
    un premier moyen de comparaison (41) comparant les valeurs calculées de la capacité de pompage du coeur gauche et/ou de la capacité de pompage du coeur droit et des valeurs de capacité de pompage cible ; et
    un premier dispositif de dosage (51) configuré pour administrer des médicaments au patient selon les résultats de comparaison dans le premier moyen de comparaison (41) ;

un deuxième moyen de calcul (32) calculant une valeur du volume de sang circulant effectif à partir de la valeur du débit cardiaque, de la valeur de la pression auriculaire gauche et de la valeur de la pression auriculaire droite qui sont entrées à partir du moyen d'entrée (2) ;

un deuxième moyen de comparaison (42) comparant la valeur du volume de sang circulant effectif calculée par le deuxième moyen de calcul (32) et une valeur de volume de sang circulant effectif cible ;

un deuxième dispositif de dosage (52) configuré pour administrer des médicaments au patient selon le résultat de comparaison dans le deuxième moyen de comparaison (42) ;

un troisième moyen de calcul (33) calculant une valeur de résistance vasculaire à partir de la valeur de débit cardiaque, de la valeur de pression auriculaire droite et de la valeur de tension artérielle qui sont entrées par le moyen d'entrée (2) ;

un troisième moyen de comparaison (43) comparant la valeur de la résistance vasculaire calculée par le troisième moyen de calcul (33) et une valeur de résistance vasculaire cible ; et

un troisième dispositif de dosage (53) configuré pour administrer des médicaments au patient selon le résultat de comparaison dans le troisième moyen de comparaison (43).

2. Système de traitement d'une maladie cardiaque selon la revendication 1, dans lequel le premier moyen de calcul (31) utilise l'équation 1 et/ou l'équation 2 pour calculer la valeur de capacité de pompage du coeur gauche et/ou la valeur de la capacité de pompage du coeur droit à partir de la valeur du débit cardiaque, la valeur de la pression auriculaire gauche et/ou la valeur de la pression auriculaire droite entrées par le moyen d'entrée (2) ;

dans lequel le deuxième moyen de calcul (32) utilise l'équation 5 pour calculer la valeur du volume de sang circulant effectif à partir de la valeur du débit cardiaque, la valeur de pression auriculaire gauche et la valeur de pression auriculaire droite entrées par le moyen d'entrée (2) ; et

dans lequel le troisième moyen de calcul (33) utilise l'équation 7 pour calculer la valeur de la résistance vasculaire à partir de la valeur du débit cardiaque, la valeur de pression auriculaire droite et la valeur de tension artérielle entrées par le moyen d'entrée (2).

Equation 1

(valeur du débit cardiaque)

= (valeur de capacité de pompage du cœur gauche) x {log ((valeur de pression auriculaire gauche) – A) + B},

où A et B sont des constantes ;

Equation 2

(valeur du débit cardiaque)

= (valeur de capacité de pompage du cœur droit) x {log ((valeur de pression auriculaire droite) – C) + D},

où C et D sont des constantes ;

Equation 5

(valeur du débit cardiaque $= \frac{1}{E}$ (valeur du volume sanguin circulant effectif)

- F (valeur de pression auriculaire droite) – G (valeur de pression auriculaire gauche),

où E, F et G sont des constantes ;

Equation 7

(valeur de la résistance vasculaire)

= {(valeur de tension artérielle) − (valeur de pression auriculaire droite) − H} /

(valeur du débit cardiaque)

où H est une constante.

3. Système de traitement d'une maladie cardiaque selon la revendication 1 ou la revendication 2, comprenant en outre :

un premier moyen de décision cible (71) calculant une valeur de capacité de pompage cible du coeur gauche et/ou une valeur de capacité de pompage cible du coeur droit à partir d'une valeur du débit cardiaque cible, d'une valeur de pression auriculaire gauche cible, et/ou d'une valeur de pression auriculaire droite cible ;
un deuxième moyen de décision cible (72) calculant une valeur de volume de sang circulant effectif cible à partir d'une valeur du débit cardiaque cible, d'une valeur de pression auriculaire gauche cible, et une valeur de pression auriculaire droite cible ;
et
un troisième moyen de décision cible (73) calculant une valeur de résistance vasculaire cible à partir d'une valeur du débit cardiaque cible, d'une valeur de pression auriculaire droite cible et d'une valeur de pression artérielle cible.

4. Système de traitement d'une maladie cardiaque selon la revendication 3, dans lequel le premier moyen de décision cible (71) utilise l'équation 3 et l'équation 4 pour calculer la valeur de capacité de pompage cible du coeur gauche et/ou la valeur de capacité de pompage cible du coeur droit à partir de la valeur du débit cardiaque cible, la valeur de pression auriculaire gauche cible et/ou la valeur de pression auriculaire droite cible ;
dans lequel le deuxième moyen de décision cible (72) utilise l'équation 6 pour calculer la valeur du volume de sang circulant effectif cible à partir de la valeur du débit cardiaque cible, la valeur de pression auriculaire gauche cible et la valeur de pression auriculaire droite cible ; et
dans lequel le troisième moyen de décision cible (73) utilise l'équation 8 pour calculer la valeur de résistance vasculaire cible à partir de la valeur du débit cardiaque cible, la valeur de pression auriculaire droite cible et la valeur de la tension artérielle cible :

Equation 3

(valeur de capacité de pompage cible du cœur gauche)

= (valeur du débit cardiaque cible) / {log((valeur de pression auriculaire gauche cible)

− A) + B}

où A et B sont des constantes ;

Equation 4

(valeur de capacité de pompage cible du cœur droit)

= (valeur du débit cardiaque cible) / {log((valeur de pression auriculaire droite cible)

− C) + D}

où C et D sont des constantes ;

Equation 6

(valeur du volume de sang circulant effectif cible)

= {(valeur du débit cardiaque cible) + F (valeur de pression auriculaire droite cible) +

G (valeur de pression auriculaire gauche cible)} x E,

où E, F et G sont des constantes ;

Equation 8

(valeur de résistance vasculaire cible)

= {(valeur de tension artérielle cible) – (valeur de pression auriculaire droite cible) –

H / (valeur du débit cardiaque cible),

où H est une constante.

5. Système de traitement d'une maladie cardiaque selon l'une quelconque des revendications 1 à 4, comprenant en outre :

un moyen d'affichage (6) affichant en continu chacune des valeurs calculées, calculées par le moyen de calcul (31), (32) et (33) dans un ordre chronologique.

6. Système de traitement d'une maladie cardiaque selon l'une quelconque des revendications 1 à 5, dans lequel la valeur du débit cardiaque est mesurée par un cathéter de Swan-Ganz ou calculée à partir d'une constante de temps diastolique d'une forme d'onde de tension artérielle.

7. Système de traitement d'une maladie cardiaque selon l'une quelconque des revendications 1 à 6, dans lequel la valeur de pression auriculaire gauche est mesurée directement par un cathéter ou est estimée en continu à partir de valeurs de pression diastolique de la pression capillaire pulmonaire bloquée ou de la tension artérielle pulmonaire qui sont mesurées par un cathéter de Swan-Ganz.

FIG. 1

FIG. 2

HEART

CARDIAC OUTPUT---TO WHOLE BODY

TO LUNGS

LEFT ATRIUM
(LEFT ATRIAL PRESSURE)

RIGHT ATRIUM
(RIGHT ATRIAL PRESSURE)

LEFT VENTRICLE

RIGHT VENTRICLE

FIG. 3

CARDIAC OUTPUT

CARDIAC OUTPUT CURVE (a)

CARDIAC OUTPUT CURVE
OF FAILING HEART (b)

LEFT ATRIAL PRESSURE

RIGHT ATRIAL PRESSURE

FIG. 4

VENOUS RETURN SURFACE OF PATIENT
WITH INCREASED EFFECTIVE CIRCULATING BLOOD VOLUME

VENOUS RETURN SURFACE

CARDIAC OUTPUT

LEFT ATRIAL PRESSURE

RIGHT ATRIAL PRESSURE

FIG. 5

CARDIAC OUTPUT CURVE (e)

VENOUS RETURN SURFACE (f)

CARDIAC OUTPUT

LEFT ATRIAL PRESSURE

RIGHT ATRIAL PRESSURE

**FIG. 6**

S1 — MEASURE ARTERIAL BLOOD PRESSURE VALUE, CARDIAC OUTPUT VALUE, AND RIGHT AND LEFT ATRIAL PRESSURE VALUES

S2 — FIRST CALCULATION MEANS CALCULATES PUMPING ABILITY VALUE OF THE LEFT HEART

S3 — SECOND CALCULATION MEANS CALCULATES EFFECTIVE CIRCULATING BLOOD VOLUME VALUE

S4 — THIRD CALCULATION MEANS CALCULATES VASCULAR RESISTANCE VALUE

S01 — FIRST TARGET DECISION MEANS CALCULATES TARGET PUMPING ABILITY VALUE OF THE LEFT HEART

S02 — SECOND TARGET DECISION MEANS CALCULATES TARGET EFFECTIVE CIRCULATING BLOOD VOLUME

S03 — THIRD TARGET DECISION MEANS CALCULATES TARGET VASCULAR RESISTANCE VALUE

S5 — DISPLAY MEANS DISPLAYS CALCULATION RESULTS OF EACH CALCULATION MEANS

S6 — FIRST COMPARISON MEANS COMPARES PUMPING ABILITY VALUE OF THE LEFT HEART

S7 — SECOND COMPARISON MEANS COMPARES EFFECTIVE CIRCULATING BLOOD VOLUME VALUE AND TARGET VALUE

S8 — THIRD COMPARISON MEANS COMPARES VASCULAR RESISTANCE VALUE AND TARGET VALUE

S9 — FIRST DOSING MEANS ADMINISTERS DRUGS ACCORDING TO COMPARISON RESULT

S10 — SECOND DOSING MEANS ADMINISTERS DRUGS ACCORDING TO COMPARISON RESULT

S11 — THIRD DOSING MEANS ADMINISTERS DRUGS ACCORDING TO COMPARISON RESULT

S5 — DISPLAY MEANS DISPLAYS CALCULATION RESULTS OF EACH CALCULATION MEANS

S12 — DISPLAY MEANS DISPLAYS DOSES OF EACH DOSING MEANS

## FIG. 7

HORIZONTAL STRAIGHT LINES INDICATE
PREDETERMINED TARGET NORMAL VALUE

ONSET OF TREATMENT

ARTERIAL BLOOD PRESSURE (mmHg)

RIGHT ATRIAL PRESSURE (mmHg)

LEFT ATRIAL PRESSURE (mmHg)

CARDIAC OUTPUT (mL/min/kg)

(MINUTE)

FIG. 8

ONSET OF TREATMENT

HORIZONTAL STRAIGHT LINES INDICATE
PREDETERMINED TARGET NORMAL VALUE

EFFECTIVE CIRCULATING BLOOD VOLUME
(mL/kg)

PUMPING ABILITY OF THE LEFT HEART
(mL/min/kg)

PUMPING ABILITY OF THE RIGHT HEART
(mL/min/kg)

VASCULAR RESISTANCE
(mmHg*kg*min/mL)

DOSE OF INOTROPIC AGENT
(dobutamine)

DOSE OF VASODILATOR
(nitroprusside)

INFUSION RATE
10mL/min

0          (MINUTE)          65

EP 2 020 248 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP TOKUHYOU2000507129 B
- US 20030199813 A
- US 20030149450 A

**Non-patent literature cited in the description**

- Determination of cardiac output by equating venous return curves with cardiac response curves. *Physiol Rev,* 1955, vol. 35, 123-129
- A novel frame work of circulatory equilibrium. *(Am) Physiol Heart Circ Physiol,* 05 February 2004, vol. 286 (6), H2376-H2385
- **Shoukas AA.** Carotid sinus baroreceptor reflex control and epinephrine. Influence on capacitive and resistive properties of the total pulmonary vascular bed of the dog. *Circ Res,* 1982, vol. 51, 95-101